# EUROPEAN PATENT APPLICATION

(11) **EP 3 479 699 A1**
(43) Date of publication of application: **08.05.2019**
(21) Application number: 17199841.2
(22) Date of filing: 03.11.2017
(51) Int. Cl.: A23C 9/20, A23C 21/00, A23L 33/00, A23L 33/135

(54) **A COMPOSITION AND USES THEREOF**

(71) Applicant: Agriculture and Food Development Authority (TEAGASC), Co. Carlow (IE)
(72) Inventor: The designation of the inventor has not yet been filed
(74) Representative: Carmody, Mark

(57) **Abstract**

A colostrum-enriched composition comprising at least 0.1 to 12 wt% immunoglobulin (at least 0.1 to 12g per 100 g composition dry weight) isolated from whey.

## Description

### Field of the Invention

The invention relates to a composition comprising components isolated from whey colostrum, and from the whey fraction of milk, and uses thereof, for example, to improve gut health in a subject and prevent and/or treat infection and gut-related diseases.

### Background to the Invention

The surface of the gastrointestinal tract is constantly exposed to microorganisms and the composition of these microbes can influence health. While there is growing recognition that diet influences the composition of the gut microbiota, few studies have explored the means by which diet can modulate the intestinal cell surface and, ultimately, shift the composition of microbial populations to facilitate improvements in health.

Bovine colostrum consists of a wide range of bioactive components including milk fat globule proteins, glycomacropeptide, α-lactalbumin (a-lac), β-lactoglobulin (β-lg), immunoglobulins and oligosaccharides each giving way to different biological functions. β-lactoglobulin's biological function is still unclear but it is known to interact with a wide range of hydrophobic ligands, while α-lactalbumin derived peptides display antimicrobial and immunomodulatory activity. Lactoferrin displays bifidogenic activity towards certain strains of *Bifidobacterium longum* subsp. *infantis, Bifidobacterium longum, Bifidobacterium bifidum* and *Bifidobacterium breve,* while the immunoglobulins of bovine colostrum confer passive immunity even in other species including humans resulting in protection of the gastrointestinal tract.

Bifidobacterium are the most dominant family in the infant intestine and confer a protective role within the infant in respect to blocking pathogen adhesion and thus preventing infection. Breast-fed infants possess a microbiota dominated by *Bifidobacterium* spp., with *Bifidobacterium infantis* and *Bifidobacterium bifidum* being the most dominant. *Bifidobacterium* spp. are particularly important for inhibiting the growth of pathogenic organisms, modulating mucosal barrier function and promoting immunological and inflammatory responses. Low abundance or absence of these species are observed in the formula-fed infant compared to breast-fed infants. These differences in the microbiota of the infant intestine have been attributed to different health outcomes in breast-fed infants, where lesser rates of infection and allergy/disease occur, while increases in cognitive function are observed in comparison with their formula-fed peers.

Angeloni *et al.* (Angeloni S, Ridet JL, Kusy N, Gao H, Crevoisier F, Guinchard S, Kochhar S, Sigrist H, Sprenger N. 2005. Glycoprofiling with micro-arrays of glycoconjugates and lectins. Glycobiology. 2005 Jan;15(1):31-41) observed that a common milk component 3'-sialyllactose exposed to the intestinal surface of Caco-2 cells induced an alteration to the Caco-2 cell surface and, subsequently, this alteration led to a 50% reduction in adhesion of enteropathogenic *Escherichia coli* (EPEC, a pathogenic strain).

It is an object of the current invention to overcome at least one of the above-mentioned problems.

### Summary of the Invention

The Applicant investigated the effect of selected milk components on modulating the intestinal cells to allow enhanced colonisation of health-promoting bacteria. After the intestinal cells were exposed to a bovine colostrum fraction, the adherence of a range of commensal bacteria to the cells was dramatically improved (up to 43-fold) when compared to the non-treated control. Overall, the colostrum fraction altered the cell surface sugar pattern thereby allowing more beneficial bacterial such as Bifidobacteria to attach to the cells. The results provide an insight into how these bacteria colonise the human gut and highlights the potential of colostrum and milk components as functional ingredients that can potentially increase commensal numbers in individuals with lower counts of health promoting bacteria such as formula-fed infants, the elderly and those on antibiotic treatment.

The invention provides an enriched whey colostrum fraction composition or an enriched whey (milk) fraction composition that comprises at least 2-12 wt% IgG (at least 2-12g per 100g of composition dry weight), at least 1-5 wt% α lactalbumin (at least 1-5g per 100g of composition dry weight) and at least 1-15 wt% β lactoglobulin (at least 1-15g per 100g of composition dry weight). Preferably, the invention provides an enriched whey colostrum fraction composition that comprises at least 2-12 wt% IgG (at least 2-12g per 100g of composition dry weight), at least 1-3 wt% α lactalbumin (at least 1-3g per 100g of composition dry weight) and at least 1.5-9.1 wt% β lactoglobulin (at least 1.5-9g per 100g of composition dry weight). The composition has been found to increase commensal colonisation in the gut by modulating the intestinal cell surface. The enriched composition described herein can be used as a supplementation in infant formula or given freely as supplements to infants. The enriched whey colostrum fraction composition described herein may also improve the discrepancy of Bifidobacterium counts found between breast-fed and formula-fed infants. Not wanting to be bound by any one particular theory, the enriched composition described herein primes the intestinal cell surface to create a more favourable environment/phenotype on the intestinal surface for commensal colonisation. This should increase commensal colonization in infants (first stage formula application) resulting in a microbiota platform closer to that of breast-fed infants.

Bovine whey colostrum and its derived components IgG, α-lactalbumin and β-lactoglobulin have not been shown previously to have the ability to alter the intestinal surface, which subsequently leads to increased commensal colonisation. IgG has been mostly associated with vaccination and combatting pathogenic infection. The colostrum-enriched composition described herein offers a different application for whey colostrum, and its components IgG, α-lactalbumin, and 3-lactoglobulin, in increasing commensal colonisation in the infant and adult gastrointestinal tract.

According to the present invention, there is provided, as set out in the appended claims, an enriched whey colostrum fraction composition comprising lactose, oligosaccharides and immunoglobulin G (IgG), which is substantially free of caseins, and wherein the IgG is at a concentration of about 0.1-12 wt% (of the composition).

Preferably, the concentration of IgG is about 0.1 wt% to 9 wt%. More preferably, the concentration of IgG is about 0.1 wt% to 2.5 wt%. Ideally, the concentration of IgG is about 0.12 wt% to 1.6 wt%. Preferably, the IgG is glycosylated.

Preferably, the composition further comprises at least about 1 wt% to 3.5 wt% α-lactalbumin or at least about 1.5 wt% to 9.1 wt% β-lactoglobulin, or a combination thereof.

Ideally, the colostrum fraction is defatted.

Preferably, the colostrum is derived from a human, or domestic animals selected from cow, sheep, goat, camel, donkey. Ideally, the colostrum is derived from bovine colostrum.

Preferably, the colostrum fraction is diluted by a factor 1:50 to 1:100. Ideally, the dilution factor is 1:70, 1:80 or 1:90. When the colostrum fraction is diluted, the concentration of IgG, α-lac and β-lg is (i) for 1:70- 0.12%(w/w), 0.13%(w/w), 0.04%(w/w), respectively; (ii) for 1:80- 0.1% (w/w), 0.11%(w/w), 0.0375%(w/w), respectively; and (iii) for 1:90- 0.1% (w/w), 0.10%(w/w), 0.03% (w/w), respectively.

There is also described an enriched whey colostrum fraction composition which when in a dry form comprises 65-75 % (w/w) total protein (including 20-30% (w/w) BSA, 2-12% IgG (w/w), 1-5% (w/w) α-lac, 1-15% (w/w) β-lg)), 0.1-1.0 % (w/w) fat, 3-13 % (w/w) inorganic constituents (*i.e.* minerals) and 16-25% (w/w) carbohydrate content, wherein the dry form components in combination amount to 100% (w/w).

Preferably, the composition in dry form comprises: 71.04 % (w/w) total protein (including 26.96% (w/w) BSA, 9% IgG (w/w), 3% (w/w) α-lac, 9.1% (w/w) β-lg)), 0.6 % (w/w) fat, 8 % (w/w) inorganic constituents (i.e. minerals) and 19.7% (w/w) carbohydrate content.

There is also described a composition comprising lactose, oligosaccharides and immunoglobulin G (IgG), which is free of caseins, and a pharmaceutical carrier or excipient, wherein the IgG is at a concentration of about 0.1-12 wt% (of the composition). Preferably, the composition is dried.

There is also described an enriched whey (milk) fraction composition comprising lactose, oligosaccharides and immunoglobulin G (IgG), which is free of caseins, and wherein the IgG is at a concentration of about 0.1-12 wt% (of the composition).

Preferably, the concentration of IgG in the composition or enriched whey (milk) fraction composition is about 0.1 wt% to 2.5 wt%. More preferably, the concentration of IgG is about 0.12 wt% to 1.6 wt%. Ideally, the IgG is glycosylated.

Preferably, the composition or enriched whey (milk) fraction composition further comprises at least about 1 wt% to 3.5 wt% α-lactalbumin or at least about 1.5 wt% to 9.1 wt% β-lactoglobulin, or a combination thereof.

There is also provided an enriched whey (milk) fraction composition comprising lactose, oligosaccharides and at least 0.1-12 wt% immunoglobulin G (IgG) (of the composition), and which is substantially free of caseins, the method comprising the steps of: defatting and de-caseinating milk; providing a milk stream that is substantially free of casein and is substantially fat-free, while retaining lactose, glycoproteins and oligosaccharides; and freeze-drying the defatted and de-caseinated milk stream.

There is also described an infant formula comprising the enriched whey colostrum fraction composition or the enriched whey (milk) fraction composition described above.

There is also described a supplement for infant formula comprising the enriched whey colostrum fraction composition or the enriched whey (milk) fraction composition described above.

There is also provided an enriched whey colostrum fraction composition as described above and obtained by a method of
defatting and de-caseinating a whey colostrum fraction;
providing a colostrum stream that is substantially free of casein and is fat-free; and
freeze-drying the defatted and de-caseinated whey colostrum fraction.

Preferably, the enriched whey colostrum fraction composition obtained by the method described above is further enriched by, for example, membrane filtration with a high molecular weight cut-off to remove proteins and peptides that are smaller in size than the IgG, as well as removing free sugars.

Preferably, the IgG is at a concentration of about 0.1-12 wt% (of the composition).

There is also provided an enriched whey colostrum fraction composition or an enriched whey (milk) fraction composition described above for use in increasing commensal colonisation of the gastrointestinal tract.

There is also provided an enriched whey colostrum fraction composition or the enriched whey (milk) fraction composition described above for use in reversing imbalances in gut microbial populations.

There is also provided an enriched whey colostrum fraction composition or the enriched whey (milk) fraction composition described above for use in a method of treating or preventing diseases associated with lower counts of commensal bacteria such as observed in formula fed infants, elderly or immune-compromised individuals or individuals on antibiotics.

Preferably, the subject is suffering from inflammatory bowel diseases (such as Crohn's disease, irritable bowel disease, ulcerative colitis), periodontal disease, rheumatoid arthritis, atherosclerosis, allergy, multi-organ failure, asthma, and allergic diseases (such as allergic rhinitis (hay fever), food allergy, and atopic dermatitis (eczema)).

The invention also relates to an enriched whey colostrum fraction composition, or an enriched whey (milk) fraction composition, or a food product of the invention, or an infant formula of the invention, for use in a method of increasing the levels of commensal bacteria in the gastrointestinal system of a mammal, especially an infant human, an adolescent human or an adult human. The invention relates to methods of treating ailments associated with lower counts of commensal bacteria, such as inflammatory bowel diseases (such as Crohn's disease, irritable bowel syndrome, ulcerative colitis), periodontal disease, rheumatoid arthritis, atherosclerosis, allergy, multi-organ failure, asthma, and allergic diseases (such as allergic rhinitis (hay fever), food allergy, and atopic dermatitis (eczema)). The method comprises administering to a subject in need thereof the enriched whey colostrum fraction composition or the (milk) composition described herein.

Typically, the colostrum (and colostrum whey fraction) is derived from a mammal. Typically, the whey (milk) fraction is derived from the milk of a mammal. Ideally, the mammal is a higher mammal, such as a human, or a domestic mammal such as a cow, a sheep, a goat, a camel, a donkey, or the like.

There is also provided a food product comprising the enriched whey colostrum fraction composition or the enriched whey (milk) fraction composition described above.

Preferably, the food product is a dairy product, a beverage, infant food, a cereal, a biscuit, confectionary, a cake, a food supplement, a dietary supplement. Ideally, the dairy product is a yoghurt, a milk drink, a flavoured milk drink, a probiotic drink, ice cream, frozen yoghurt. Preferably the food product is an infant formula.

Preferably, the food product comprises from 0.1-50 wt% of the enriched whey colostrum fraction composition or enriched whey (milk) fraction composition.

The enriched whey (milk) fraction composition can be in a wet or a dry form, for example, a particulate product such as a powder, flakes, pellets and the like. The dry enriched whey (milk) fraction composition may be generated by means of any suitable drying technique, such as spray drying, drum drying or freeze-drying. The invention also relates to liquid enriched whey (milk) fraction compositions comprising the milk composition of the invention suspended or solubilised in a suitable liquid such as water.

The composition is preferably in dry form, for example a particulate product such as a powder, flakes, pellets and the like. The dry composition may be generated by means of any suitable drying technique, such as spray drying, drum drying or freeze-drying. The invention also relates to liquid compositions comprising the composition of the invention suspended or solubilised in a suitable liquid such as water.

There is also provided a method for producing an enriched whey colostrum fraction composition comprising lactose, oligosaccharides and at least 0.1-12 wt% immunoglobulin G (IgG) (of the composition), and which is substantially free of caseins, the method comprising the steps of:
defatting and decaseinating a colostrum fraction;
providing a colostrum stream that is substantially free of casein, fat-free and retains lactose, glycoproteins and oligosaccharides; and
freeze-drying the defatted and de-caseinated whey colostrum fraction.

There is also provided an enriched whey colostrum fraction composition described herein for use in a method of treating or preventing diseases associated with lower counts of commensal bacteria such as inflammatory bowel diseases (such as Crohn's disease, irritable bowel syndrome, ulcerative colitis), periodontal disease, rheumatoid arthritis, atherosclerosis, allergy, multi-organ failure, asthma, and allergic diseases (such as allergic rhinitis (hay fever), food allergy, and atopic dermatitis (eczema)).

There is also provided a composition for use in increasing adherence of commensal bacteria in the gut, the composition comprising at least one of glycosylated IgG, glycans, monosaccharides, or a combination thereof.

Preferably, the glycosylated IgG is at a concentration of about 0.1-12 wt% (of the composition).

Preferably, the glycan comprises sialic acid, *N*-Acetylglucosamine, fucose, mannose, galactose, fructose, glucose, or a combination thereof.

Preferably, the composition is in dry form, for example a particulate product such as a powder, flakes, pellets and the like. The dry composition may be generated by means of any suitable drying technique, such as spray drying, drum drying or freeze-drying. The invention also relates to liquid compositions comprising the composition of the invention suspended or solubilised in a suitable liquid such as water.

Preferably, the composition also comprises a pharmaceutically acceptable carrier.

Ideally, the composition is presented as a food product, such as a dairy product, a beverage, infant food, a cereal, a biscuit, confectionary, a cake, a food supplement, a dietary supplement. Ideally, the dairy product is a yoghurt, a milk drink, a flavoured milk drink, a probiotic drink, ice cream, frozen yoghurt. Preferably the food product is an infant formula.

### Definitions

In this specification, the term "enriched whey colostrum composition" should be understood to mean a composition derived from a whey colostrum fraction which when in a dry form comprises 65-75 % (w/w) total protein (including 20-30% (w/w) BSA, 2-12% IgG (w/w), 1-5% (w/w) α-lac, 1-15% (w/w) β-lg)), 0.1-1.0 % (w/w) fat, 3-13 % (w/w) inorganic constituents (*i.e.* minerals) and 16-25% (w/w) carbohydrate content, wherein the dry form components in combination amount to 100% (w/w). Ideally, the composition in dry form comprises: 71.04 % (w/w) total protein (including 26.96% (w/w) BSA, 9% IgG (w/w), 3% (w/w) α-lac, 9.1% (w/w) β-lg)), 0.6 % (w/w) fat, 8 % (w/w) inorganic constituents (i.e. minerals) and 19.7% (w/w) carbohydrate content. The quantity of BSA, α-lac, β-lg and IgG are percentage of dry form powder and not of protein.

The term "colostrum-derived" should be understood to mean that the composition is obtained from a milk colostrum fraction (CF). When bovine colostrum is used, the isolated fraction for use in the composition is termed bovine whey colostrum fraction (BWCF).

The term "colostrum" should be understood to be the form of milk produced by a mammal within the first seven days of giving birth.

In the specification, the term "enriched whey fraction composition" should be understood to mean a composition sourced from the whey fraction of milk which when in a dry form comprises 65-75 % (w/w) total protein (including 20-30% (w/w) BSA, 2-12% IgG (w/w), 1-5% (w/w) α-lac, 1-15% (w/w) β-lg)), 0.1-1.0 % (w/w) fat, 3-13 % (w/w) inorganic constituents (*i.e.* minerals) and 16-25% (w/w) carbohydrate content, wherein the dry form components in combination amount to 100% (w/w). Ideally, the composition in dry form comprises: 71.04 % (w/w) total protein (including 26.96% (w/w) BSA, 9% IgG (w/w), 3% (w/w) α-lac, 9.1% (w/w) β-lg)), 0.6 % (w/w) fat, 8 % (w/w) inorganic constituents (i.e. minerals) and 19.7% (w/w) carbohydrate content. The quantity of BSA, α-lac, β-lg and IgG are percentage of dry form powder and not of protein.

The term "defatting" or "defatted" should be understood to mean removal of the fat component of colostrum or milk via centrifugation, which results in a substantially fat-free enriched whey colostrum fraction. The term "substantially fat-free" should be understood to mean the enriched whey colostrum fraction having trace amounts of fat, that is, having a fat content of between 0.1-1.0 % (w/w).

The term "de-caseinated" or "de-caseinating" should be understood to mean removal of casein from the colostrum fraction by centrifugation following treatment with 1M HCI and heat. This process leaves the colostrum fraction substantially free or completely free of casein. Removal of casein reduces the complexity of whey colostrum by removing high molecular weight proteins such as casein and albumin, and by doing so, increases the concentration and exposure of the less abundant proteins that exist in the colostrum.

The term "substantially free" should be understood to mean where the enriched colostrum fraction or composition described herein is free from casein, or has a trace amount of casein, such as 0.01-0.5 wt% casein, that does not have any influence on the efficacy of the enriched whey colostrum fraction.

In the specification, the units "wt%" and "w/w" should be understood to mean Xg per 100g of the composition and can be used interchangeably.

The term "inorganic constituents" should be understood to mean minerals, such as calcium, sodium, potassium and chloride.

The term "carbohydrate content" should be understood to mean the glycan portion of a glycoconjugate, such as a glycoprotein, a glycolipid, or a proteoglycan, as well as lactose and oligosaccharides that may be present.

The term "glycosylation" should be understood to mean the reaction in which a carbohydrate, *i.e.,* a glycosyl donor (such as a sugar unit), is attached to a hydroxyl or other functional group of another molecule (a glycosyl acceptor, such as a protein).

The term "glycan(s)" should be understood to mean compounds consisting of a number of monosaccharides linked glycosidically. Glycans typically are a saccharide(s) that can be attached 1) to each other through glycosidic linkages, and/or 2) to a wide variety of biological molecules through glycosylation to augment their function. Glycans can be attached to another molecule via N-linkages or O-linkages. In the case of N-glycans, glycans are attached to the amine group of asparagine in the sequence Asn-X-Ser/Thr, where X represents any amino acid except proline. All N-glycans share a common core sugar sequence, Manα1-6(Manα1-3)Manβ1-4GlcNAcβ1-4GlcNAcβ1-Asn-X-Ser/Thr. All O-glycans are assembled one sugar at a time on a serine or threonine residue of a peptide chain.

In the specification, the term "free glycan" should be understood to mean glycans which are free in solution or have been cleaved or released from glycosylated IgG and are free in solution or are free in the colostrum fraction/whey (milk) fraction.

In the specification, the term "monosaccharide" should be understood to mean a sugar molecule having a chemical formula: Cx(H₂O)y, where conventionally x ≥ 3 and/or derivatives of amine containing monosaccharides.

The term "infant food" should be understood to mean food which is processed and manufactured to meet the nutritional requirements of infants within their first year of life, such as infant formula, follow-on formula and starting solid infant food.

In a specific embodiment, the term "pharmaceutically acceptable" means approved by a regulatory agency of the Federal or a state government or listed in the U.S. Pharmacopeia or other generally recognized pharmacopeia for use in animals, and more particularly in humans.

The term "carrier" refers to a diluent, adjuvant, excipient, or vehicle with which the Therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsion, tablets, pills, capsules, powders, sustained-release formulations and the like.

The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the patient. The formulation should suit the mode of administration.

### Brief Description of the Drawings

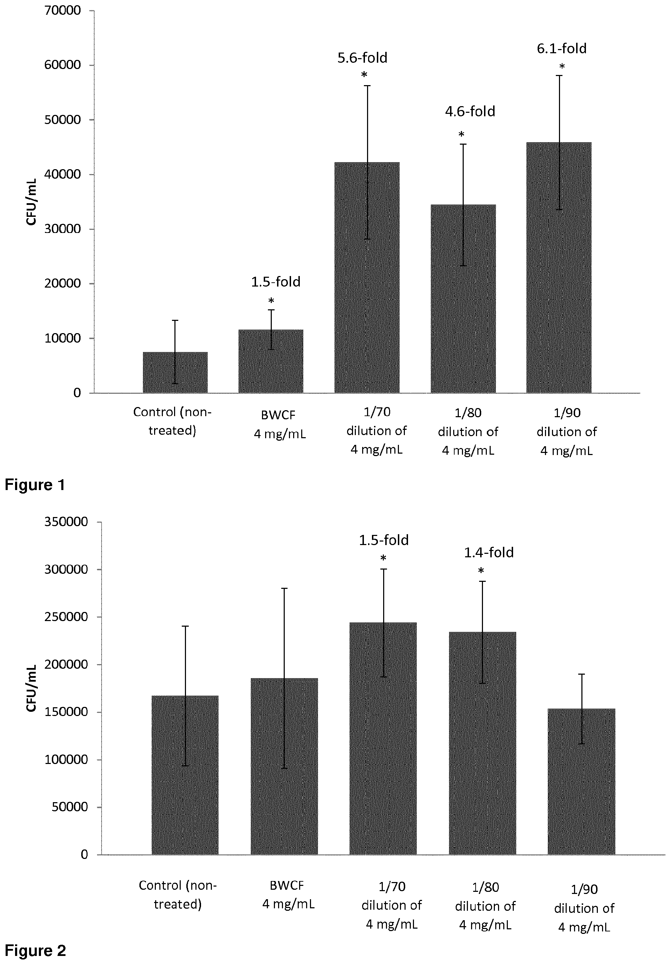

The invention will be more clearly understood from the following description of an embodiment thereof, given by way of example only, with reference to the accompanying drawings, in which:-
**Figure 1** is a bar chart illustrating a 1.5 to 6.1-fold increase in *Bifidobacterium longum NCIMB 8809* adherence to HT29 cells after exposure to various dilutions of the BWCF
**Figure 2** is a bar chart illustrating a 1.4 to 1.5-fold increase in *Bifidobacterium longum subsp. infantis ATCC* 15697 adherence to HT29 cells after exposure to various dilutions of the BWCF

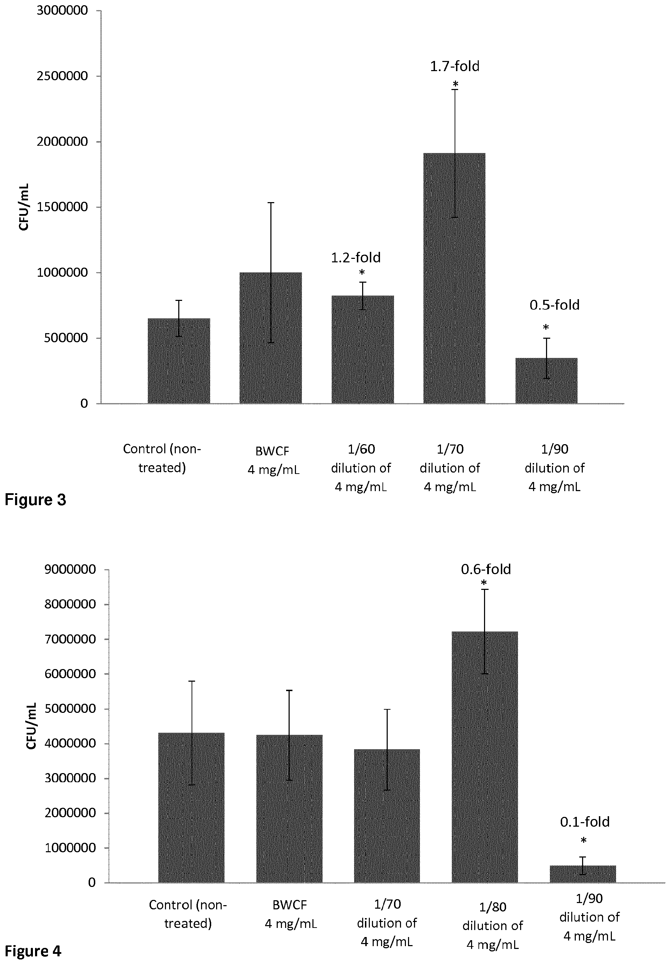

**Figure 3** is a bar chart illustrating 0.5 to 1.7-fold increase in *Bifidobacterium breve* 2258 adherence to HT29 cells after exposure to various dilutions of the BWCF
**Figure 4** is a bar chart illustrating a 0.1 to 1.6-fold increase in *Bifidobacterium breve* UCC2003 adherence to HT29 cells after exposure to various dilutions of the BWCF

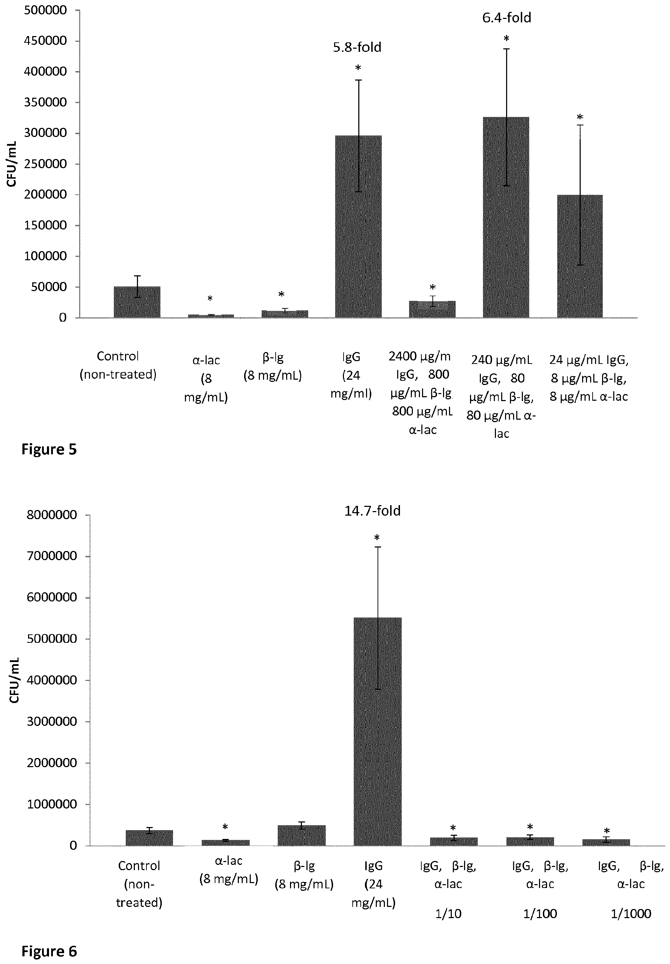

**Figure 5** is a bar chart illustrating a 5.8 to 6.4-fold increase in *Bifidobacterium longum* NCIMB 8809 adherence to HT-29 cells after prior exposure of HT-29 cells to individual components of the whey colostrum; α-lactalbumin (α-lac), 3-lactoglobulin (β-Ig), IgG, as well as mixtures thereof. There was a 5.8 to 6.4-fold increase in *Bifidobacterium longum* NCIMB 8809 adherence to HT-29 cells after exposure to IgG and a combination of IgG, β-lg and α-lac.
**Figure 6** is a bar chart illustrating *Bifidobacterium longum* subsp. *infantis* 15697 adherence to HT-29 cells after prior exposure of HT-29 cells to individual components; α-lactalbumin, 3-lactoglobulin, IgG and mixtures thereof. There was a 0.4-fold increase and a 14.7-fold increase in *Bifidobacterium longum* subsp. *infantis* 15697 adherence to HT-29 cells after prior exposure of HT-29 cells to a combination of IgG, β-lg and α-lac, and IgG alone, respectively.

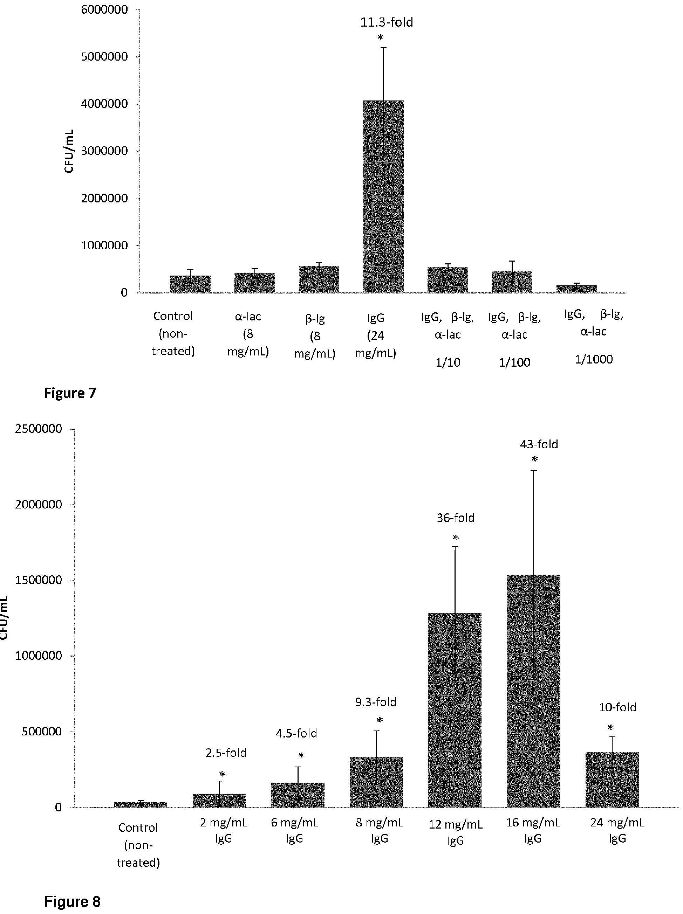

**Figure 7** is a bar chart illustrating *Bifidobacterium longum* subsp. *infantis* 2258 adherence to HT-29 cells after prior exposure of HT-29 cells to individual components; α-lactalbumin, 3-lactoglobulin, IgG and mixtures thereof. There was an 11.3-fold increase in *Bifidobacterium longum* subsp. *infantis* 2258 adherence to HT-29 cells after prior exposure of HT-29 cells to IgG alone.
**Figure 8** is a bar chart illustrating *Bifidobacterium longum* NCIMB 8809 adherence to HT-29 cells after prior exposure of HT-29 cells to purified IgG (extracted and purified from day 1 colostrum) in the concentration range 2-24 mg/mL. There was a 2.5 to 43-fold increase of *Bifidobacterium longum* NCIMB 8809 after prior exposure to the different range of IgG concentrations.

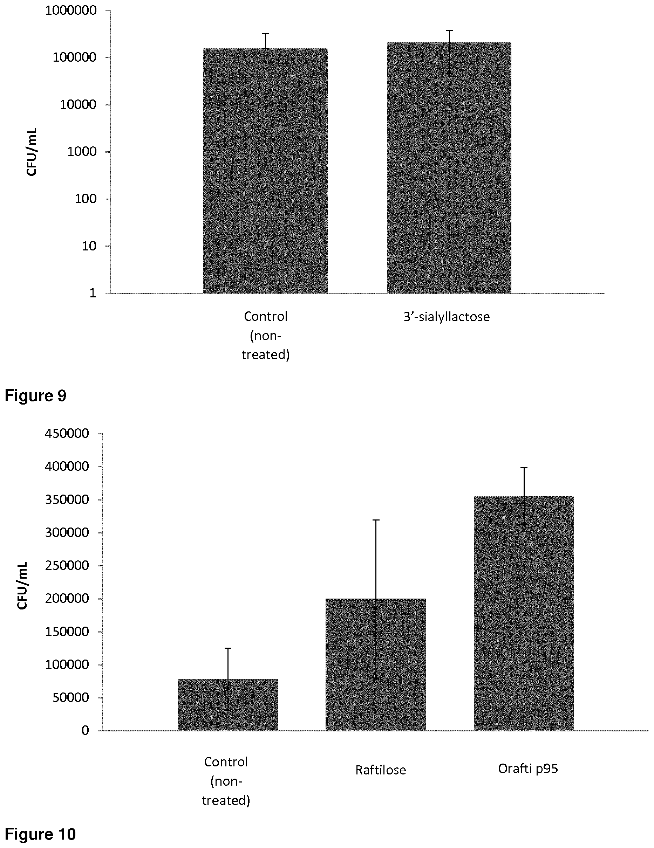

**Figure 9** is a bar chart illustrating *Bifidobacterium longum* NCIMB 8809 adherence to HT-29 cells after prior exposure of HT-29 cells to the milk oligosaccharide 3'-sialyllactose at 4 mg/mL. There was no increase in adhesion of *Bifidobacterium longum* NCIMB 8809 after prior exposure to 3'-sialyllactose.
**Figure 10** is a bar chart illustrating *Bifidobacterium longum* NCIMB 8809 adherence to HT-29 cells after prior exposure of HT-29 cells to two commercial prebiotics oligofructose (Orafti p95) (Beneo Orafti, Dublin) and an oligofructose-enriched inulin (Raftilose) at 4 mg/mL. There was no increase in adhesion of *Bifidobacterium longum* NCIMB 8809.

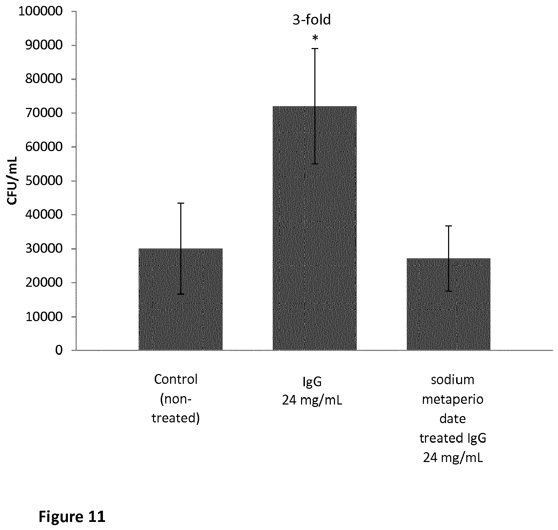

**Figure 11** is a bar chart illustrating *Bifidobacterium longum* NCIMB 8809 adherence to HT-29 cells after prior exposure of HT-29 cells to IgG and sodium metaperiodate treated IgG at 24 mg/mL. There was no increase in adhesion of *Bifidobacterium longum* NCIMB 8809 when exposed to the sodium metaperiodate-treated IgG unlike non-treated IgG which still gave a significant increase (3-fold) in adhesion of *Bifidobacterium longum* NCIMB 8809. Although not being bound by theory, this data supports the hypothesis that the glycan terminal moieties of IgG are responsible for its observed effect on HT-29 cells, which leads to HT-29 cells being better colonisers for commensal bacteria such as *Bifidobacterium longum* NCIMB 8809.

### Detailed Description of the Drawings

### Materials and Methods

### Generation of Bovine Whey Colostrum fraction (BWCF)

A fraction was isolated from bovine colostrum (Day 1) as follows:
- The colostrum as collected from Holstein Friesian cattle on-site at the Teagasc Food Research Centre, Moorepark (Fermoy, Cork, Ireland) and was defatted and deproteinized through conventional methods as described by Kobata & Ginsburg (Oligosaccharides of human milk. IV. Isolation and characterization of a new hexasaccharide, lacto-N-neohexaose. Arch Biochem Biophys 150, 273-281 (1972)).
- In brief, aliquots were centrifuged at 5000 rpm for 20 min at 4 °C in a Sorvall RC6 plus® to separate the fat.
- The aqueous phase was collected and 1 M-HCI was added until a pH of 4.6 was reached.
- The sample was then heated to 35°C for 2hr and precipitated caseins were removed by centrifugation at 5000 rpm for 30 min at 25 °C.
- The pH of the supernatant was then neutralised using 4M NaOH.
- The de-fatted, de-caseinated solution was collected, freeze-dried and stored at - 80 °C.

### Bacterial Strains Conditions

*Bifidobacterium longum* subsp. *infantis* ATCC 15697 and *Bifidobacterium adolescentis* ATCC 15703 was purchased from DSMZ (Germany). *Bifidobacterium longum* NCIMB 8809, *Bifidobacterium breve* UCC2003, *Bifidobacterium breve* NCFB 2258, and *Lactobacillus rhamnosus* GG were obtained from Teagasc Food Research Centre culture collection. The strains were stored in deMan Rogosa Sharpe (MRS) [Difco, Sparks, MD, USA] broth containing 50% glycerol at -80°C until they were re-cultured according to the supplier instructions. The strains were cultured twice in MRS media supplemented with L-cysteine (0.05%w/v) prior to use with the exception of *Lactobacillus rhamnosus* GG which was cultured twice in un-supplemented MRS. The strains were cultured overnight at 37°C under anaerobic conditions generated using a Anaerocult A system (Merck, Dannstadt, Germany). The strains were grown overnight and used at an optical density (OD600) of 0.45-0.5. To prepare mid-exponential phase cells, overnight cultures were adjusted to an optical density of 0.3 and the cultures were then grown for a further 2 hours and then adjusted to an optical density (OD600 nm) of 0.45-0.5 (corresponding to 5X107 CFU/mL).

### Cell culture and exposure of HT-29 cells to milk components

The human colon adenocarcinoma cell line HT29 was purchased from the American Type Culture Collection (ATCC). HT29 cells were routinely grown in McCoy's 5A modified medium (Sigma). All cells were routinely maintained in 75 cm² tissue culture flasks and incubated at 37°C in a humidified atmosphere (5% CO₂). Cells were passaged when the confluency of the flasks was approximately 80%. HT29 cells were seeded into 12 well plates (Corning) at a concentration of 1x10⁵ cells/well and incubated at 37°C in 5% CO₂ in a humidified atmosphere. The cells were fed every second day with McCoy's media (10% FBS) until 100% confluency was achieved (typically 3-4 days). Prior to sample exposure, the cells were washed and placed in 2% FBS McCoy's media. After 24 hours, BWCF, purified IgG, α-lac and β-lg, were re-suspended in pre-heated McCoy's 5A (0% FBS) each at given concentrations as indicated in the figures (see Figs. 1-8). The samples were then filter sterilized with a 0.2µm (individually packed sterile) filter. Non-supplemented McCoy's 5A medium was used as a non-treated control (NT). The cells were then washed with phosphate buffered saline and the samples were applied. The plates were then incubated at 37 °C incubator for 24 hours in a in a humidified atmosphere (5% CO₂) prior to bacterial exposure.

### Adhesion assays

On the day of the assay, as mentioned above, the primarily anaerobic strains were used at an optical density (OD600) of 0.45-0.5. The bacteria were then centrifuged at 5000 g for 10 minutes, after which the supernatant was removed and the pellet was washed with 0% FBS McCoy's media and centrifuged again. This wash step was repeated twice after which the bacteria was re-suspended in 0% FBS McCoy's media. Bacterial exposure to eukaryotic cells was for 2 hours at 37°C under anaerobic conditions using an Anaerocult A system (Merck, Dannstadt, Germany). The cells were then washed four times with PBS to remove non-adherent bacteria. The cells were then lysed with 500µl per well of 0.1% Triton X100; (Sigma, Steinheim, Germany) for 10 minutes at 37 °C. The lysates were serially diluted and enumerated by spread-plating on MRS plates. The bacteria cells are then spread plated onto De Man, Rogosa and Sharpe (MRS) agar plates. The plates were incubated anaerobically at 37°C in a 5% CO₂ incubator for 48 hrs. CFU were then counted. Adhesion was determined as the fold change difference between the control treated cells adherent bacteria and the treated cells adherent bacteria. Fold adhesion = [CFU/mL of recovered adherent bacteria of treated cells ÷ CFU/mL of recovered adherent bacteria of non-treated cells]. Adhesion assays were performed in triplicate.

### Periodate treatment of IgG

IgG was treated with sodium metaperiodate (NaIO4) to produce IgG-P (IgG - periodate). IgG (24 mg/mL) was incubated with 10 mM NaIO4 at room temperature for 30 min. Excess NaIO4 was removed by centrifugal filtration using 3 kDa MWCO with three phosphate buffered saline, pH 7.4 (PBS) washes and the retentate containing GMP-P was re-suspended in 0% McCoy's media. This was then exposed to HT-29 cells for 24 hours as per adhesion assay protocol.

### Statistical Analysis

Data are expressed as mean ± standard deviation (SD) of the results of three independent assays conducted in triplicate. Graphs were generated using Microsoft Excel. Where student t-tests were used p≤0.05 was considered significant.

### Experimental

The inventors investigated whether a bovine whey colostrum containing IgG, α-lactalbumin and β-lactoglobulin could alter the cell surface of HT-29 cells and make it then more favourable for colonisation by commensal strains. A de-fatted, de-caseinated bovine colostrum fraction (Day 1 colostrum) was originally generated and exposed to HT-29 cells for 24 hours. After 24 hours, the sample was washed off and different Bifidobacterial strains were introduced to the HT-29 cells. Increases in the adherence of all 7 commensal strains tested were observed ranging from 0.6-6.1-fold (Figure1-8).

The increase in attachment of the commensal strains to the epithelial cell surface is hypothesised to be related to a change in the glycosylation pattern of the epithelial cell surface induced by BWCF exposure. It was also investigated what bioactive component was being retained by the whey colostrum and found that IgG acting independently and/or synergistically with both α-lactalbumin and 3-lactoglobulin (in the case of one strain B. *longum* 8809) was the most likely candidate with the activity being concentration and strain dependent (Figure 1-8).

The data presented here shows an increase in commensal strain adherence. When the colostrum fraction is added to the HT29 cells, the fraction appears to be able to induce or cause an alteration in the HT29 cell environment allowing for better commensal strain adherence. IgG with or without α-lactalbumin and 3-lactoglobulin alters the cell surface of HT-29 cells to create a more favourable phenotype for commensal colonisation. Exposure of IgG isolated from day 1 colostrum, induced a change to the cell surface of intestinal cells. This change on the cell surface leads to a dramatic increase in the ability of infant commensal bacteria to attach to the cell, which may result in multiple health benefits to the infant.

Fermentation of inulin-type fructans selectively stimulate growth of *Bifidobacteria,* and thus increase the number of potentially health-promoting bacteria and reduce the number of potentially harmful species. Both oligofructose and inulin are prebiotic. Lactoferrin has shown to retain bifidogenic activity of certain strains of *Bifidobacterium longum* subsp. *infantis, Bifidobacterium longum, Bifidobacterium bifidum* and *Bifidobacterium breve.* Compounds which are known to elicit bifidogenic activity such as oligosaccharides, lactoferrin, an oligofructose-enriched inulin *etc.* do not induce the observed effect of enhanced adhesion (see Figures 9 and 10), hence demonstrating this activity occurs through a non-prebiotic mechanism. BWCF with minimal processing has shown to alter the HT-29 cell surface to increase counts of beneficial bacteria. Thus, the ability to alter the intestinal surface environment is unique to the colostrum fraction.

A sample of IgG isolated from the bovine whey colostrum fraction was treated with sodium metaperiodate (a reagent used to open saccharide rings, basically cleave all glycans away). The treated IgG was exposed to HT-29 cells and was found that the treated IgG did not induce the same effect on *Bifidobacterium* (*i.e.* no increased adhesion) when compared to non-treated IgG (see Figure 11). This suggests that the glycans of IgG are responsible for the observed effect of increased adhesion.

Bovine whey colostrum and its derived components IgG, α-lactalbumin and β-lactoglobulin have not been shown previously to have the ability to alter the intestinal surface, which subsequently leads to increased commensal colonisation. IgG has been mostly associated with vaccination and combatting pathogenic infection. This invention offers a different application for whey colostrum and its components IgG, α-lactalbumin and β-lactoglobulin in increasing commensal colonisation in the infant and adult gastrointestinal tract.

Bovine milk is used as the main understudy for human milk and most infant formulae are based on bovine milk. IgG is found in large amounts in bovine colostrum (46.40 g/L) and it is the dominant immunoglobulin in bovine colostrum and milk. The whey colostrum has also been tested with two adult strains (Bifidobacterium adolescentis ATCC 15703 and Lactobacillus rhamnosus GG) also giving rise to increased adhesion *in vitro,* suggesting applications in foods other than infant formula, possibly as a supplement for adults suffering from gut dysbiosis.

In conclusion, IgG (preferably glycosylated) with or without α-lactalbumin,β-lactoglobulin, and/or free glycans, alters the cell surface of HT-29 cells to create a more favourable phenotype for commensal colonisation. Exposure of intestinal cells to IgG isolated from day 1 colostrum, induced a change to the cell surface of intestinal cells. This change on the cell surface leads to a dramatic increase in the ability of infant commensal bacteria to attach to the cell, which may result in multiple health benefits to the infant. This should increase commensal colonization in infants (first stage formula application) resulting in a microbiota platform closer to that of breast-fed infants. Also, gut dysbiosis can cause many chronic inflammatory diseases, such as inflammatory bowel disease, obesity, cancer, and autism. It could be associated with ailments which are associated with lower counts of commensal bacteria such as asthma, allergic disease and bowel problems.

In the specification, the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. An enriched whey colostrum fraction composition comprising lactose, oligosaccharides and immunoglobulin G (IgG), which is substantially free of caseins, and wherein the IgG is at a concentration of about 0.1-12 wt% (of the composition).

2. An enriched whey colostrum fraction composition of Claim 1, further comprising at least about 1 wt% to 3.5 wt% α-lactalbumin or at least about 1.5 wt% to 9.1 wt% β-lactoglobulin, or a combination thereof.

3. An enriched whey colostrum fraction composition of Claim 1 or Claim 2, wherein the IgG is glycosylated.

4. An enriched whey colostrum fraction composition of any one the preceding Claims, wherein the colostrum is defatted.

5. An enriched whey colostrum fraction composition according to any one of the preceding claims, wherein the colostrum is derived from a human, or domestic animals selected from cow, sheep, goat, camel, donkey.

6. An enriched whey colostrum composition according to any one of the preceding claims, in which the colostrum is diluted by a factor 1:50 to 1:100.

7. An enriched whey colostrum fraction composition which when in a dry form comprises 65-75 % (w/w) total protein (including 20-30% (w/w) BSA, 2-12% IgG (w/w), 1-5% (w/w) α-lac, 1-15% (w/w) β-Ig)), 0.1-1.0 % (w/w) fat, 3-13 % (w/w) inorganic constituents and 16-25% (w/w) carbohydrate content, wherein the dry form components in combination amount to 100% (w/w).

8. An enriched whey colostrum fraction composition of Claim 7, wherein the composition in dry form comprises: 71.04 % (w/w) total protein (including 26.96% (w/w) BSA, 9% IgG (w/w), 3% (w/w) α-lac, 9.1% (w/w) β-lg)), 0.6 % (w/w) fat, 8 % (w/w) inorganic constituents and 19.7% (w/w) carbohydrate content.

9. An enriched whey (milk) fraction composition comprising lactose, oligosaccharides, immunoglobulin G (IgG), and which is free of caseins, and wherein the IgG is at a concentration of about 0.1-12 wt% (of the milk composition).

10. An enriched whey (milk) fraction composition of Claim 9, wherein the IgG is glycosylated.

11. An infant formula comprising the enriched whey colostrum fraction composition of any one of Claims 1 to 8 or the enriched whey (milk) fraction composition according to any one of Claims 9 or 10.

12. A method for producing an enriched whey colostrum fraction composition comprising lactose, oligosaccharides and at least 0.1-12 wt% immunoglobulin G (IgG) (of the composition), and which is substantially free of caseins, the method comprising the steps of:
(a) defatting and de-caseinating a whey colostrum fraction;
(b) providing a colostrum stream that is substantially free of casein and is substantially fat-free, while retaining lactose, glycoproteins and oligosaccharides; and
(c) freeze-drying the defatted and de-caseinated whey colostrum fraction.

13. An enriched whey colostrum fraction composition according to any of Claims 1 to 8, or an enriched whey (milk) fraction composition according any one of Claims 9 to 10, for use in a method of treating or preventing diseases associated with lower counts of commensal bacteria such as observed in formula fed infants, elderly or immune-compromised individuals or individuals on antibiotics.

14. A composition for use in increasing adherence of commensal bacteria in the gut, the composition comprising at least one of glycosylated IgG, glycans, monosaccharides or a combination thereof.

15. A composition according to Claim 14, wherein the glycan comprises sialic acid, N-Acetylglucosamine, fucose, mannose, galactose, fructose, glucose, or a combination thereof.
